(19) Europäisches Patentamt
European Patent Office
Office européen des brevets

(11) **EP 2 243 423 B1**

(12) **EUROPÄISCHE PATENTSCHRIFT**

(45) Veröffentlichungstag und Bekanntmachung des Hinweises auf die Patenterteilung:
**02.05.2012 Patentblatt 2012/18**

(21) Anmeldenummer: **09450088.1**

(22) Anmeldetag: **23.04.2009**

(51) Int Cl.:
*A61B 5/00* *(2006.01)*    *A61M 5/172* *(2006.01)*

(54) **Verfahren und Gerät zur Ermittlung von Empfehlungen für Wirkstoffdosierungen anhand von Messserien zumindest eines physiologischen Parameters eines Patienten**

Method and device for determining recommendations for dosing agents on the basis of measurement series of at least one physiological parameter of a patient

Procédé et appareil de détermination de conseils pour dosages de matière active à l'aide de séries de mesures d'au moins un paramètre physiologique d'un patient

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**

(43) Veröffentlichungstag der Anmeldung:
**27.10.2010 Patentblatt 2010/43**

(73) Patentinhaber:
• **Roche Diagnostics GmbH**
**68305 Mannheim (DE)**
Benannte Vertragsstaaten:
**DE**
• **F. Hoffmann-La Roche AG**
**4070 Basel (CH)**
Benannte Vertragsstaaten:
**AT BE BG CH CY CZ DK EE ES FI FR GB GR HR HU IE IS IT LI LT LU LV MC MK MT NL NO PL PT RO SE SI SK TR**

(72) Erfinder:
• **Krainz, Michael**
**8043 Graz (AT)**
• **Leiner, Marco Jean-Pierre**
**8045 Graz (AT)**

(74) Vertreter: **Margotti, Herwig Franz**
**Schwarz & Partner**
**Patentanwälte**
**Wipplingerstrasse 30**
**1010 Wien (AT)**

(56) Entgegenhaltungen:
WO-A-2008/057213    WO-A-2009/049245
GB-A- 2 436 873    US-A- 5 697 899
US-A1- 2007 168 136    US-A1- 2009 054 753

• **MATHIJS VOGELZANG ET AL: "Computer-assisted glucose control in critically ill patients" INTENSIVE CARE MEDICINE, SPRINGER, BERLIN, DE, Bd. 34, Nr. 8, 4. April 2008 (2008-04-04), Seiten 1421-1427, XP019619075 ISSN: 1432-1238**
• **PAUL C DAVIDSON ET AL: "A computer-directed intravenous insulin system shown to be safe, simple, and effective in 120,618 h of operation" DIABETES CARE, AMERICAN DIABETES ASSOCIATION, ALEXANDRIA, VA, US, Bd. 20, Nr. 10, 1. Januar 2005 (2005-01-01), Seiten 2418-2423, XP009120784 ISSN: 0149-5992**
• **BODE ET AL: "Intravenous Insulin Infusion Therapy: Indications, Methods, and Transition to Subcutaneous Insulin Therapy" ENDOCRINE PRACTICE, AMERICAN ASSOCIATION OF CLINICAL ENDOCRINOLOGY, US, Bd. 10, Nr. Suppl.2, 1. Januar 2004 (2004-01-01), Seiten 71-80, XP009120785 ISSN: 1530-891X**

**Beschreibung**

Die vorliegende Erfindung betrifft ein Verfahren zur Durchführung einer Serie von Messungen zumindest eines physiologischen Parameters, insbesondere Blutwertes, wie z.B. Blutzucker, an Proben, die einem Patienten zu diskreten Messzeitpunkten entnommen werden, und zur Errechnung einer Empfehlung für eine Dosierung zumindest eines dem Patienten bis zum nächsten Messzeitpunkt zu verabreichenden Wirkstoffs anhand eines den zumindest einen gemessenen physiologischen Parameter einbeziehenden Dosierungsvorschlagsalgorithmus, wodurch zumindest ein physiologischer Parameter des Patienten auf einen Zielbereich eingestellt bzw. in diesem Zielbereich gehalten wird.

Die Erfindung betrifft weiters ein medizinisch-diagnostisches Analysegerät zur Durchführung des obigen Verfahrens.

Bei vielen medizinischen Behandlungen von Patienten ist es erforderlich, gewisse physiologische Parameter des Patienten regelmäßig zu überwachen und mittels Wirkstoffgabe in einen definierten Wertebereich zu bringen bzw. in diesem Wertehreich zu halten. Beispielsweise kann Hyperglykämie (d.h. zu hohe Blutzuckerwerte (über 110 mg/dl)) an Patienten postoperativ in der Intensivstation auch bei Nichtdiabetikern auftreten. Eine Normalisierung des Blutzuckerspiegels durch ständige Glukosemessung in Verbindung mit gezielter Insulingabe (Tight Glycemic Control) in dieser Phase führt zu einem signifikanten Abnehmen der Sterblichkeitsrate. Dieser Zusammenhang wurde 2001 erstmals in einer Studie erwähnt und seither mehrfach bestätigt. Es wurden bereits computer-implementierte Algorithmen entwickelt, die das Spitalspersonal bei der Dosierung der Insulingabe unterstützen. Ein solcher Algorithmus, der sich in der Praxis gut bewährt hat, wurde als "Glucommander"-Algorithmus von Forschern von Atlanta Diabetes Associates entwickelt und ist z.B. in dem Artikel "Intravenous Insulin Infusion Therapy: Indications, Methods, and Transition to Subcutaneous Insulin Therapy", Bode et al, ENDOCRINE PRACTICE, Vol 10 (Suppl 2) March/April 2004 sowie in einem Artikel von Davidson et al. in Diabetes Care, Vol. 20, Nr. 10, 2418-2423, 2005 beschrieben. Die Prinzipien des "Glucommandet"-Algorithmus lassen sich in einem kartesischen Koordinatensystem mit dem Blutzuckerspiegel als Abszisse und einer Insulindosis [Einheiten pro Stunde] als Geradenbündel darstellen, wobei jeden Gerade einen unterschiedlichen Multiplikator repräsentiert. Senkrechte Linien in diesem Diagramm definieren einen Blutzucker-Bereich, in den der Patient gebracht werden soll bzw. in dem er gehalten werden soll. Die Multiplikatorlinien weisen darauf hin, wie schnell die Veränderung des Blutzuckerspiegels erfolgen soll, in anderen Worten, wie hoch die Insulindosis bis zum nächsten Messzeitpunkt zu wählen ist. Nach jeder neuen Messung des Blutzuckerspiegels erfolgt eine Neubewertung der zu verabreichenden Wirkstoffdosis, wobei ein Wechsel des Multiplikators vorgenommen werden kann. Die Messung des Blutzuckerspiegels kann mit einem handelsüblichen Blutglukosemessgerät, beispielsweise einem speziellem Blutglukosemessgerät oder auch einem Multiparametermessgerät wie beispielsweise einem Blutgasanalysator zur Bestimmung der Blutgase, Elektrolyte und Metabolite (Glukose, Laktat) vorgenommen werden.

Der "Glucommander"-Algorithmus hat sich zur Unterstützung des Pflegepersonals in der Intensivmedizin bewährt. Eine Voraussetzung für seine erfolgreiche Anwendung ist jedoch, dass die vorgeschriebenen Intervalle zwischen zwei Blutzuckerspiegel-Messungen exakt eingehalten werden. Dies kann aber aus verschiedenen Gründen nicht garantiert werden, sondern in der Praxis existieren Messausschlusszeitfenster, in denen keine Messungen möglich sind. Solche Messausschlusszeitfenster können messgerätebedingt auftreten, beispielsweise, wenn das Messgerät eine periodische Kalibrierung oder sonstige interne Wartungs- und Prüfvorgänge durchlaufen muss. Messausschlusszeitfenster können auch benutzerbedingt auftreten, beispielsweise, wenn ein Patient für gewisse Zeiten nicht für eine Blutzuckerspiegel-Messung zur Verfügung steht, weil er andere Untersuchungen oder Verrichtungen zu erledigen hat. Wenn Messausschlusszeitfenster mit vorgeschriebenen Zeitpunkten für Blutzuckerspiegel-Messungen zusammenfalten, wird die Empfehlung des "Glucommander"-Algorithmus suboptimal, eventuell sogar für den Patienten riskant. Blutzuckerwerte eines Patienten, insbesondere im postoperativen Bereich, nicht mehr kontrollieren zu können, erhöht stark das Risiko für das Auftreten von Hyper- oder Hypoglykämien.

Ein System und ein Verfahren gemäß des des Oberbegriffs des Anspruchs 1 ist beispielsweise aus WO 2009/049245 bekannt. Obwohl der Messzyklus zu anderen als den geplanten Zeitpunkten beginnen kann, wird eine Berücksichtigung von geräte-und/oder benutzerbedingten Messausschlusszeitfenstern gemäß der Definition des kennzeichnenden Teils des Anspruchs 1 jedoch nicht offenbart.

Die Aufgabe der Erfindung besteht nun darin, eine Lösung für das Problem zu finden, dass von einem zu überwachenden Patienten physiologische Parameter in einer Messserie erfasst werden müssen, um auf Basis dieser Messserie unter Anwendung eines Dosierungsvorschlagsalgorithmus eine Empfehlung für eine Dosierung eines dem Patienten bis zum nächsten Messzeitpunkt zu verabreichenden Wirkstoffs zu errechnen, wobei die empfohlene Dosierung auch dann nicht das gesundheitliche Risiko für den Patienten erhöhen darf, wenn die vorgesehenen Messzeitpunkte in Messausschlusszeitfenster fallen. Insbesondere besteht die Aufgabe der Erfindung darin, zu vermeiden, dass mit den wie oben beschrieben ermittelten Empfehlungswerten Blutzuckerwerte nicht mehr kontrolliert werden können und damit das Risiko für das Auftreten von Hyper- oder Hypoglykämien steigt, wenn die empfohlenen Messzeitpunkte nicht eingehalten werden können.

Die vorliegende Erfindung löst die gestellte Aufgabe durch das Verfahren mit den Merkmalen des Anspruchs 1 sowie durch Bereitstellen eines medizinisch-diagnostischen Analysegeräts mit den Merkmalen des Anspruchs. 11. Vorteilhafte Ausgestaltungen und Fortbildungen der Erfindung sind in den Ünteranspruchen dargelegt.

Das erfindungsgemäße Verfahren umfasst das Durchführen einer Serie von Messungen zumindest eines physiologischen Parameters, insbesondere Blutwertes, wie z.B. Blützucker, eines Patienten, wobei dem Patienten zu diskreten Messzeitpunkten Proben entnommen werden, an denen die Messungen durchgeführt werden. Aus den Messungen wird eine Empfehlung für eine Dosierung zumindest eines dem Patienten bis zum nächsten Messzeitpunkt zu verabreichenden Wirkstoffs ermittelt, wozu ein den zumindest einen gemessenen physiologischen Parameter einbeziehender Dosierungsvorschlagsalgorithmus angewandt wird. Dadurch ist es möglich, zumindest einen physiologischen Parameter des Patienten auf einen Zielbereich einzustellen bzw. in dem Zielbereich zu halten. Es sei erwähnt, dass der zumindest eine physiologische Parameter des Patienten, der auf einen Zielbereich eingestellt wird bzw, darin gehalten wird, nicht notwendigerweise jener physiologische Parameter ist, der in den Proben gemessen wird. Es liegt vielmehr auch im Rahmen der vorliegenden Erfindung, indirekte Messungen durchzuführen, d.h. einen physiologischen Parameter zu messen, der mit dem einzustellenden physiologischen Parameter in einem Zusammenhang steht und sich bei Wirkstoffverabreichung in einer mit dem einzustellenden physiologischen Parameter zusammenhängen.den Weise verändert. Der Dosierungsvorschlagsalgorithmus ist so ausgelegt, dass der nächste Messzeitpunkt unter Berücksichtigung von vorbestimmten Messausschlusszeitfenstern ermittelt und der verantwortlichen Person mitgeteilt wird. Der ermittelte nächste Messzeitpunk stellt eine Variable des Dosierungsvorschlagsalgorithmus der Erfindung dar, d.h. die Errechnung der vorgeschlagener Wirkstoffdosis erfolgt unter Berücksichtigung veränderter Zeitintervalle, zwischen den Messungen oder/und unter Neuberechnung des bei der nächsten Messung zu erwartenden Werts des zumindest einen physiologischen Parameters.

Der Begriff "Wirkstoff" ist so zu verstehen, dass er auch jegliche pharmazeutische präparation umfasst, die diesen Wirkstoff enthält.

Es liegt auch im Umfang der Erfindung dass eine Vielzahl physiologischer Parameter gemessen werden, die der Dosierungsvorschlagsalgorithmus zur Berechnung der Dosierungsempfehlung für einen Wirkstoff heranzieht. Es ist an sich bekannt, mehrere physiologische Parameter zur Berechnung einer Dosierung bzw. Dosierempfehlung eines Wirkstoffs heranzuziehen, siehe z.B. US 2007/0168136 A.

In einer vorteilhaften Ausgestaltung der Erfindung bezieht der Dosierungsvorschlagsalgorithmus von einem Benutzer eingegebene Patientendaten, wie Gewicht, Ernährungsgewohnheiten, etc. bei der Errechnung der Empfehlung ein. Diese Maßnahme ist an sich bekannt, siehe z.B. WO 2008 057213, in der offenbart ist, dass mehrere physiologischer Parameter (z.B. Gewicht, Körpertemperatur) zur Errechnung einer . Dosierung eines Wirkstoffs (Insulin) herangezogen werden. Dabei ist zu unterscheiden zwischen bekannten, voreinstellbaren Parametern, wie dem Gewicht des Patienten, und physiologische Parametern, die laufend gemessen werden. Beide Gruppen von physiologischen Parametern können vom Dosierungsvorschlagsalgorithmus für die Berechnung einer Empfehlung einer Wirkstoffverabreichung herangezogen werden.

In einer Ausführungsform der Erfindung wird der nächste Messzeitpunkt ermittelt, indem zum letzten Messzeitpunkt ein Zeitintervall addiert und geprüft wird, ob der sich daraus ergebende vorläufige nächste Messzeitpunkt in einem Messausschlusszeitfenster liegt und, falls zutreffend, der nächste Messzeitpunkt außerhalb des Messausschlusszeitfensters verlegt wird. Bei dieser Ausführungsform braucht man nicht beachten, ob das Messausschlusszeitfenster analysegerätbedingt oder benutzerbedingt auftritt. Um eine mögliche Gesundheitsgefährdung des Patienten aufzuschließen, ist vorgesehen, dass die Verlegung des nächsten Messzeitpunkts außerhalb des Messausschlusszeitfensters unter Vorausberechnung eines zu erwartenden Messwerts und einer Risikoabschätzung, ob der zu erwartende Messwert für den Zustand des Patienten akzeptabel ist, erfolgt. Alternativ dazu kann die Verlegung des nächsten Messzeitpunkts außerhalb des Messausschlusszeitfensters unter Risikoabschätzung einer für den Zustand des Patienten maximal zulässigen Zeitspanne zwischen zwei Messzeitpunkten erfolgen. Sollte sich bei den oben genannten Risikoabschätzungen ein unvertretbar hohes Risiko ergeben, so wird an den Benutzer eine Warninformation ausgegeben, wobei optional der Messzeitpunkt vor das Messausschlusszeitfenster verlegt wird.

Falls das Messausschlusszeitfenster messgerätebedingt ist, so ist in einer Ausführungsform der Erfindung vorgesehen, dass, falls der berechnete vorläufige nächste Messzeitpunkt in dem Messausschlusszeitfenster liegt, das messgerätebedingte Messausschlusszeitfenster außerhalb des nächsten Messzeitpunkts verlegt wird. Diese Ausführungsform bietet den Vorteil, dass die Messungen und Dosierungsempfehlungen wie geplant weitergeführt werden können. Falls das Messausschlusszeitfenster benutzerbedingt ist, so ist in einer Ausführungsform der Erfindung vorgesehen, dass, falls der berechnete vorläufige nächste Messzeitpunkt in dem Messausschlusszeitfenster liegt, dem Benutzer vorgeschlagen wird, seine zum Messausschlusszeitfenster führenden Aktionen so zu verlegen, dass sie nicht mit den errechnete nächsten Messzeitpunkten kollidieren. Der Benutzer kann daraufhin das benutzerbedingte Messausschlussfenster außerhalb des nächsten Messzeitpunkts verlegen, und gegebenenfalls auch nachfolgende benutzerbedingte Messausschlussfenster verlegen.

Diese Ausführungsform bietet den Vorteil, dass die Messungen und Dosierungsempfehlungen wie ursprünglich geplant weitergeführt werden können.

Ein medizinisch-diagnostisches Analysegerät zur Durchführung des erfindungsgemäßen Verfahrens, das beispielsweise als Blutanalysegerät ausgebildet ist, umfasst zumindest einen Messsensor zur Messung zumindest eines physiologischen Parameters an den Proben, die einem Patienten zu diskreten Messzeitpunkten entnommen werden. In einer Ausgestaltung des Analysegeräts ist zumindest eine Probenaufnahme zur Aufnahme der von einem Patienten zu diskreten Messzeitpunkten entnommenen Proben vorgesehen, wobei der zumindest eine Messsensor zur Messung des zumindest einen physiologischen Parameters an den Proben mit der zumindest einen Probenaufnahme kommuniziert. Die Messsignale des zumindest einen Messsensors werden von einer Recheneinheit erhalten und zu Messwerten des zumindest einen physiologischen Parameters aus den Messsignalen aufbereiter. Die Messwerte werden in einem den zumindest einen physiologischen Parameter einbeziehenden Dosierungsvorschlagsalgorithmus zur Errechnung einer Empfehlung für eine Dosierung zumindest eines dem Patienten bis zum nächsten Messzeitpunkt zu verabreichenden Wirkstoffs verwendet. Diese Empfehlung sowie Warnungen und andere Meldungen werden von der Recheneinheit über ein Ausgabeinterface an einen Benutzer ausgegeben. Bevorzugt ist die Recheneinheit dazu ausgebildet, das Verfahren parallel für eine Vielzahl von Patienten durchzuführen.

Zusammengefasst bietet die Erfindung die folgenden Vorteile:

- Der nächste Messzeitpunkt wird so festgelegt, dass das Messgerät sicher messbereit ist.

- Die Höhe der zu verabreichenden Wirkstoffdosis wird entsprechend dem Verschieben des Messzeitpunkts angepasst.

- Aufgrund der Berechnung des zu erwarteten Werts des physiologischen Parameters kann auf diverse Aktionen vorzeitig reagiert werden.

- Aufgrund der Kenntnis von Geräte- und/oder Benutzeraktionen kann der nächste Messzeitpunkt und die Empfehlung für die Wirkstoffdosierung optimiert werden.

Die Erfindung wird nun anhand von Ausführungsbeispielen unter Bezugnahme auf die Zeichnung näher erläutert. In den Zeichnungen zeigen:

Fig. 1 ein Schema eines erfindungsgemäßen medizinisch-diagnostischen Analysegeräts, mit dem das erfindungsgemäße Verfahren durchgeführt wird;

Figuren 2 und 3 schematische Zeitdiagramme zur Erläuterung von Ausführungsformen des erfindungsgemäßen Verfahrens; und

Fig. 4 Blutzuckerspiegelverläufe eines Patienten über der Zeit in Abhängigkeit von Dosierungsempfehlungen.

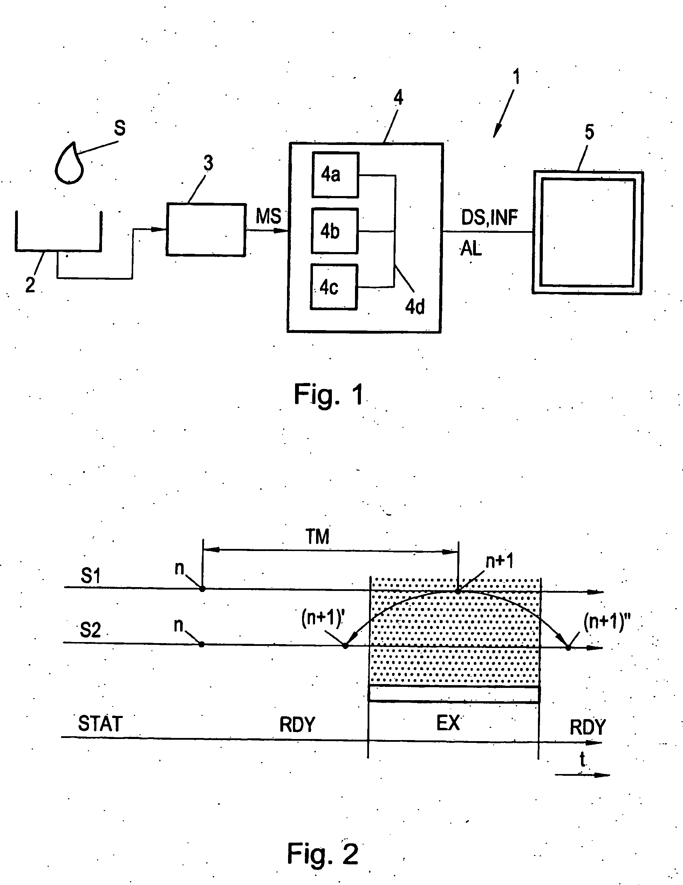

In Fig. 1 ist ein erfindungsgemäßes medizinisch-diagnostisches Analysegerät 1 schematisch in einem Blockschaltbild dargestellt. Dieses medizinisch-diagnostische Analysegerät 1 umfasst eine Probenaufnahme 2 zur Aufnahme von einem Patienten zu diskreten Messzeitpunkten entnommenen Proben S, zumindest einen mit der Probenaufnahme kommunizierenden Messsensor 3 zur Messung zumindest eines physiologischen Parameters, insbesondere Blutwertes, wie z.B. Blutzucker, an den Proben S, weiters eine die Messsignale MS des zumindest einen Messsensors 3 erhaltende Recheneinheit 4 zur Aufbereitung von Messwerten der physiologischen Parameter aus den Messsignalen MS. Aus den Messwerten errechnet die Recheneinheit 4 eine Empfehlung für eine Dosierung zumindest eines dem Patienten bis zum nächsten Messzeitpunkt zu verabreichenden Wirkstoffs anhand eines den zumindest einen gemessenen physiologischen Parameter einbeziehenden Dosierungsvorschlagsalgorithmus. Die Recheneinheit 4 umfasst zumindest einen Prozessor 4a, einen Programmspeicher 4b und einen Arbeitsspeicher 4c, die untereinander durch ein Bussystem 4d verbunden sind. Soweit bisher beschrieben kann das Analysegerät 1 auf Basis eines handelsüblichen Blutgasanalysators zur Bestimmung der Blutgase, Elektrolyte und Metabolite (Glukose, Laktat) oder eines sonstigen Blutglukosemessgerätes aufgebaut sein, wie sie von der Anmelderin erzeugt und vertrieben werden.

Das erfindungsgemäße medizinisch-diagnostische Analysegerät unterscheidet sich von den bekannten Analysatoren durch einen darin implementierten Workflow zur Durchführung von Messserien und ein Verfahren (Algorithmus) zur Errechnung einer Empfehlung einer zu verabreichenden Wirkstoffdosis zwischen zwei Messzeitpunkten. Innerhalb der Messserie werden die relevanten physiologischen Parameter diskret über die Zeit durch manuelle Probenahme und Messung bestimmt. Alternativ sind auch automatisierte Probennahme-und/oder Messschritte möglich. Der Dosierungsvorschlagsalgorithmus ist als ausführbare Programm implementiert, das im Programmspeicher 4b abgelegt ist und von der Recheneinheit 4 abgearbeitet wird. Das Ergebnis der Berechnungen der Recheneinheit 4 ist eine Dosierungsempfehlung DS an einen Benutzer des Analysegeräts 1 für eine kontinuierliche oder periodische oder einem anderen Verabreichungsprofil folgende Abgabe zumindest eines Wirkstoffes an einen Patienten (z.B. mittels Infusionen), kann aber alternativ oder zusätzlich Warnungen AL und allgemeine Meldungen INF umfassen Die Dosierungsempfehlung DS, Warnungen AL und Meldungen INF werden von der Recheneinheit 4 an ein Ausgabeinterface 5 übertragen, das z.B. als Display, Drucker, etc. realisiert ist. Das Analysegerät 1 ist so ausgelegt, dass die Recheneinheit 4 parallel für eine Vielzahl von Patienten Messserien durchführt und Wirkstoffdosierungsempfehlungen errechnet.

Zu beachten ist, dass es bei diesem Typ von medizinisch-diagnostischem Analysegerät 1 oft messgerätebedingte Messausschlusszeitfenster gibt, zu denen Geräteaktionen, wie zum Beispiel eine Systemkalibration, stattfinden müssen und zu denen infolge dessen keine Messungen durchgeführt werden können. Weiters können benutzerbedingte Messausschlusszeitfenster existieren, z.B. aufgrund arbeitsbedingter Umstände, die ebenfalls eine Messung verhindern.

Das erfindungsgemäße medizinisch-diagnostische Analysegerät 1 arbeitet solcherart, dass bei dem implementierten Dosierungsvorschlagsalgorithmus die Messausschlusszeitfenster berücksichtigt werden, wobei der ermittelte nächste Messzeitpunkt bevorzugt eine Variable des Dosierungsvorschlagsalgorithmus darstellt. Wie im Zeitdiagramm von Fig. 2 dargestellt, wird in einem ersten Schritt S1 der nächste Messzeitpunkt $n+1$ ermittelt, indem zum letzten Messzeitpunkt $n$ ein Zeitintervall TM addiert und anschließend geprüft wird, ob der sich daraus ergebende vorläufige nächste Messzeitpunkt $n+1$, in einem Messausschlusszeitfenster EX liegt. Dies ist hier der Fall und daher wird in einem Schritt S2 der nächste Messzeitpunkt außerhalb des Messausschlusszeitfensters EX in einen Zeitraum RDY verlegt, zu dem das Analysegerät 1 betriebsbereit ist, wie in der Statuszeile STAT in Fig. 2 zu sehen ist. Die Verlegung des nächsten Messzeitpunkts kann dabei vor $(n+1)'$ das Messausschlusszeitfenster EX erfolgen, oder dahinter $(n+1)''$. Bei der Verlegung des nächsten Messzeitpunkts erfolgt gegebenenfalls auch eine entsprechende Anpassung der Wirkstoffdosis. Die Entscheidung, ob die Verlegung des nächsten Messzeitpunkts vor $(n+1)'$ oder hinter $(n+1)'$ das Messausschlusszeitfenster EX erfolgen soll, kann gemäß den folgenden Fallunterscheidungen getroffen werden:

- **Fall 1:** Der berechnete nächste Messzeitpunkt $n+1$ fällt in die erste Halbzeit des Messausschlusszeitfensters EX. Dann wird der Messzeitpunkt $(n+1)'$ vor den Beginn des Messausschlusszeitfensters EX verlegt.

  Ist der eingesetzte Dosierungsempfehlungsalgorithmus so ausgelegt, dass ein bestimmtes Zeitintervall $TM_{min}$ (z.B. 15 min) zwischen aufeinanderfolgenden Messungen nicht unterschritten werden sollte, um zu verlässlichen Dosierungsempfehlungen für eine Wirkstoffgabe zu gelangen, können noch folgende speziellen Fallgestaltungen unterschieden werden:

- **Fall 1a:** Der berechnete nächste Messzeitpunkt $n+1$ fällt in die erste Halbzeit des Messausschlusszeitfensters EX und der zeitliche Abstand zwischen dem Zeitpunkt der aktuellen Messung $n$ und einem gemäß obiger Annahme vor den Beginn des Messausschlusszeitfensters EX zu verlegenden Messzeitpunkts $(n+1)'$ ist, gegebenenfalls unter Berücksichtigung der Dauer der Durchführung eines Messvorganges, kleiner als das nicht zu unterschreitende Zeitintervall $TM_{min}$. Um in diesem Fall das nicht zu unterschreitende Zeitintervall $TM_{min}$ nicht zu unterschreiten, wird in diesem Fall der nächste Messzeitpunkt $(n+1)''$ hinter das Ende des Messausschlusszeitfensters EX verlegt.

- **Fall 1b:** Der berechnete nächste Messzeitpunkt $n+1$ fällt in die erste Halbzeit des Messausschlusszeitfensters EX und der zeitliche Abstand zwischen dem Zeitpunkt der aktuellen Messung $n$ und einem gemäß obiger Annahme vor den Beginn des Messausschlusszeitfensters EX zu verlegenden Messzeitpunkts $(n+1)'$ ist, gegebenenfalls unter Berücksichtigung der Dauer der Durchführung eines Messvorganges, größer als das nicht zu unterschreitende Zeitintervall $TM_{min}$. In diesem Fall wird der nächste Messzeitpunkt $(n+1)'$ vor den Beginn des Messausschlusszeitfensters EX verlegt.

- **Fall 2:** Der berechnete nächste Messzeitpuhkt $n+1$ fällt in die zweite Halbzeit oder exakt auf die Halbzeit des Messausschlusszeitfensters EX. Dann wird der nächste Messzeitpunkt $(n+1)''$ hinter das Ende des Messausschlusszeitfensters EX verlegt.

  Ist der eingesetzte Dosierungsempfehlungsalgorithmus so ausgelegt, dass ein bestimmtes Zeitintervall $TM_{Max}$ (z.B. 60 min) zwischen aufeinanderfolgenden Messungen nicht überschritten werden sollte, um zu verlässlichen Dosierungsempfehlungen für eine Wirkstoffgabe zu gelangen, können noch folgende speziellen Fallgestaltungen unterschieden werden:

- **Fall 2a:** Der berechnete nächste Messzeitpunkt $n+1$ fällt in die zweite Halbzeit oder exakt auf die Halbzeit des Messzeitausschlussfensters EX und der zeitliche Abstand zwischen dem Zeitpunkt der aktuellen Messung $n$ und einem gemäß obiger Annahme nach den Beginn des Messausschlusszeitfensters EX zu verlegenden Messzeitpunkts $(n+1)''$ ist, gegebenenfalls unter Berücksichtigung der Dauer der Durchführung eines Messvorganges, größer als das nicht zu überschreitende Zeitintervall $TM_{max}$. Um in diesem Fall das nicht zu überschreiten Zeitintervall $TM_{max}$ nicht zu überschreiten, wird in diesem Fall der nächste Messzeitpunkt $(n+1)'$ vor den Beginn des Messausschlusszeitfensters EX verlegt.

- **Fall 2b:** Der berechnete nächste Messzeitpunkt $n+1$ fallt in die zweite Halbzeit oder exakt auf die Halbzeit des Messzeitausschlussfenstets EX und der zeitliche Abstand zwischen dem Zeitpunkt der aktuellen Messung $n$ und einem gemäß obiger Annahme nach den Beginn des Messausschlusszeitfensters EX zu verlegenden Messzeitpunkts $(n+1)''$ ist, gegebenenfalls unter Berücksichtigung der Dauer der Durchführung eines Meßvorganges, kleiner als das nicht zu überschreitende Zeitintervall $TM_{max}$. In diesem

Fall wird der nächste Messzeitpunkt (n+1)" hinter das Ende des Messausschlusszeitfensters EX verlegt.

Solche minimalen oder maximalen Zeitintervalle zwischen aufeinanderfolgenden Messzeitpunkten können insbesondere dann relevant werden, wenn gewährleistet sein sollte, dass aufeinanderfolgende Messzeitpunkte in möglichst regelmäßigen Abständen liegen, um eine möglichst optimale Einstellung des Patienten auf einen bestimmten Zielwert eines physiologischen Parameters, z.B. des Blutzuckerwertes, zu ermöglichen.

Bei der Berechnung der möglichen Verlegung des Messzeitpunkts bezieht ein solcher möglicher Dosierungsvorschlagsalgorithmus die folgenden Aspekte ein:

• Der Abstand zwischen der letzten Messung (n) und einer nach hinten verlegten Messung (n+1)" gewährleistet die Anforderung an das Risiko.

• Der zu erwartende vorausberechnete Messwert des physiologischen Parameters gewährleistet die Anforderung an.das Risiko.

Bei Vor- oder Rückverlegen des nächsten Messzeitpunkts kann der erwartete Messwert der Folgemessung des physiologischen Parameters vorausberechnet und in Abhängigkeit davon die empfohlene Wirkstoffdosis verändert werden, falls der vorausberechnete Messwert die Anforderung an das Risiko nicht gewährleistet, wie nachfolgend erläutert wird.

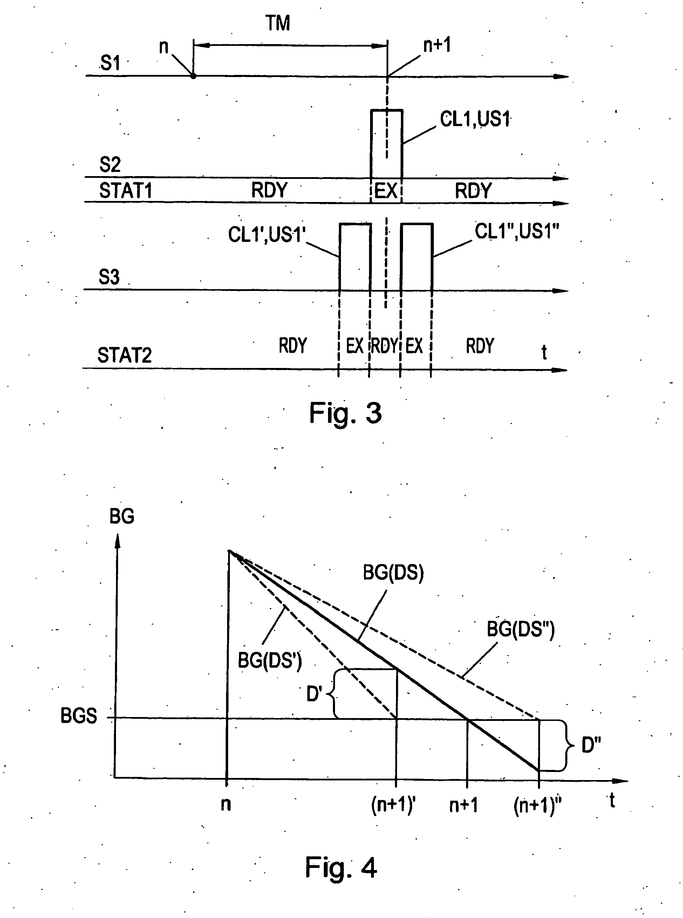

Fig. 4 zeigt ein beispielhaftes Diagramm eines Blutzuckerspiegels BG eines Patienten über der Zeit t. Zum Messzeitpunld n erstellt der Dosierungsvorschlagsalgorithmus eine Dosierungsempfehlung DS zur Abgabe eines Wirkstoffs (in diesem Beispiel Insulin) an den Patienten. Die Dosierungsempfehlung DS ist so bemessen, dass der Verlauf des Blutzuckerspiegels BG(DS) zum Zeitpunkt n+1 der nächsten Messung einen Sollblutzuckerspiegel BGS erreichen sollte. Falls es nötig ist, den Zeitpunkt der nächsten Messung aufgrund von Messausschlusszeitfenstern nach vorne (n+1)' oder hinten (n+1)" zu verlegen, so wird sich zum vorverlegten Messzeitpunkt (n+1)' eine Abweichung D' vom Sollblutzuckerspiegel BGS nach oben bzw. zum rückverlegten Messzeitpunkt (n+1)" eine Abweichung D" nach unten einstellen. Der Dosierungsvorschlagsalgorithmus kann nun mittels Interpolation einerseits diese Abweichungen zum Zeitpunkt (n+1)', (n+1)" berücksichtigen, indem er als Sollwert des Blutzuckerspiegels die Differenzwerte berücksichtigt und bei einer Berechnung einer neuen Dosierungsempfehlung von diesen abweichenden Werten ausgeht. Er führt weiters eine Risikoabschätzung durch, ob die Abweichungen, speziell bei nach hinten verlegten Messzeitpunkten, eventuell so groß sind, dass Komplikationen für den Patienten zu berücksichtigen sind und daher die Messzeitpunktverlegung aus Sicherheitsgründen inakzeptabel ist und der Benutzer gewarnt werden muss. Der Dosierungsvorschlagsalgorithmus kann aber auch die geänderten Messzeitpunkte (n+1)', (n+1)" insoweit berücksichtigen, dass er geänderte Dosierungsempfehlungen DS', DS" abgibt, die beim Patienten zu Blutzuckerspiegelverläufen BG(DS'), BG(DS") führen, die ein Erreichen des Sollblutzuckerspiegels BGS zu den geänderten Messzeitpunkten (n+1)', (n+1)" ermöglichen.

Anhand von Fallbeispielen werden nun weitere Ausführungsvarianten der Erfindung erläutert, wobei stets in einem Schritt S1 ein Zeitpunkt für die nächste Messung n+1 berechnet und dann geprüft wird, ob der berechnete nächste Messzeitpunkt n+1 in ein Messausschlusszeitfenster EX fällt, zu dem keine Messung durchgefüm werden kann, sei es gerätebedingt oder benutzerbedingt.

Fig. 3 zeigt ein Zeitdiagramm, bei dem Messausschlusszeitfenster durch Aktionen CL1, des Analysegeräts 1 auftreten, z.B. durch interne Kalibrierungsvorgänge, oder durch Aktionen US1 eines Benutzers auftreten. Wie aus der Zeile S2 und der Statuszeile STAT1 ersichtlich, würden die Aktionen CL1, US1 des Analysegeräts 1 bzw. des Benutzers mit dem sich aus dem Zeitabstand TM zum vorhergehenden Messzeitpunkt n errechneten Zeitpunkt n+1 der nächsten Messung .zusammenfallen, d.h, ein Messausschlusszeitfenster EX definieren, das den Messzeitpunkt n+1 inkludiert. Um dies zu vermeiden, werden in einem Schritt S3 die geplanten Aktionen CL1 bzw. US1 verlegt, und zwar nach entweder nach vorne (CL1', US1') oder nach, hinten (CL1", US1"). Dies erfolgt unter Berücksichtigung der Gewährleistung der Anforderungen an das Risiko. Wie aus der Statuszeile STAT2 ersichtlich, fallen somit die sich aus der Verlegung der Aktionen des Analysegeräts ergebenden Messausschlusszeitfenster EX in Zeiten, die nicht mit dem geplanten Zeitpunkt n+1 der nächsten Messung zusammenfallen. Das heißt, das Analysegerät 1 ist zum geplanten Messzeitpunkt n+1 messbereit (RDY).

Eine weitere Ausführungsform der Erfindung betrifft den Fall, dass das Messausschlusszeitfenster zu groß ist, um risikolos eine Verlegung des nächsten Messzeitpunkts durchzuführen. In diesem Fall verlegt der Algorithmus den Zeitpunkt für die nächste Messung vor das Messausschlusszeitfenster und gibt eine Warnung AL an den Benutzer aus, dass das Erreichen oder Aufrechterhalten der Zielwerte des physiologischen Parameters nicht gewährleistet ist und der Benutzer eigene Maßnahmen setzen muss.

Nachfolgend wird der Ablauf des erfindungsgemäßen Verfahrens für das Anwendungsbeispiel der Überwachung von Blutwerten, insbesondere des Blutzuckerspiegels, von Patienten unter Verwendung des oben erläuterten Analysegeräts 1 beschrieben. Bei diesem Anwendungsfall werden die Blutzuckerwerte einer Vielzahl von Patienten im Point of Care Bereich (insbesondere Intensivstation) parallel, durch jeweils eine eigene Messserie je Patient, mittels manuellen Blutzuckermessungen überwacht. Nach jeder Blutzuckermessung wird für jeden Patienten durch einen entsprechenden Algorithmus eine Insulindosis empfohlen, welche bis zum nächsten Messzeitpunkt zu verabreichen ist.. Ein erhöhter Glukosespiegel soll durch eine, mittels Dosierpumpe dauerhaft an den Patienten verabreichte Insulindosis gesenkt und innerhalb eines definierten Zielbereiches stabilisiert werden. Der Zeitpunkt der nächsten Messung wird zusammen mit der Insulindosis bestimmt und angezeigt. Weiters soll bei Erreichen dieses Zeitpunkts ein stiller Alarm (Anzeige) ausgelöst werden.

In den Einstellungen des Analysegeräts 1 können global (d.h, einheitlich für alle Patienten):

- das maximale Intervall zwischen zwei Messungen innerhalb einer Messserie und
- der Maximalwert der zu berechnenden Insulindosis

eingestellt werden.

In den Einstellungen des Analysegeräts 1 können weiters individuell (d.h. für jeden Patienten einzeln) Patientendaten, wie Geburtsdatum, Gewicht, Ernährungsgewohnheiten, Insulinsensitivitätsfaktoren etc., die der Dosierungsvorschlagsalgorithmus bei der Errechnung der Empfehlung (DS) für die Insulindosierung einbeziehen soll, eingestellt werden.

Eine Messserie wird während der Messung nach Eingabe der Patienten Identifikation (ID) gestartet. Das Startverhalten des Dosierungsvorschlagsalgorithmus (mild, normal, benutzerdefiniert), ein Startmultiplikator (0.5 bis 2.0) und ein Glukose-Zielwert (oder Zielbereich) können für jeden Patienten individuell bei der ersten Messung festgelegt werden.

Die Blutentnahme (venös oder arteriell) erfolgt manuell mit konventionellen Probenahmegefäßen (Spritzen, o.ä.). Das Probenahmegefäß wird manuell mit dem Analysegerät 1 in Kontakt gebracht und die Messung gestartet, wonach automatisch zumindest ein Aliquot der Probe in das Analysegerät 1 eingesaugt wird und wonach die Messung erfolgt. Sobald der Glukosewert am Analysegerät 1 gemessen ist, berechnet der implementierte Algorithmus auf Basis des gemessenen Glukosewertes die notwendige Insulindosis, dies dem Patienten bis zum nächsten Messzeitpunkt dauerhaft mittels einer Dosierpumpe verabreicht werden soll. Der Zeitpunkt der nächsten Messung wird zusammen mit der Insulindosis angezeigt.

Die Daten werden in der Datenbank des Analysegeräts 1 abgespeichert und gegebenenfalls an ein LIS/HIS (Krankenhausinformationssystem) übertragen.

Der Benutzer dosiert die Insulingabe am Patienten mittels Dosierpumpe und muss die tatsächlich verabreichte Insulindosis am Analysegerät spätestens zu Beginn der nächstfolgenden Messung derselben Messserie bestätigten.

Optional kann der Benutzer dem Patienten eine geänderte Insulindosis verabreichen und muss diese, zusammen mit einem Kommentar am Analysegerät 1 bestätigen.

In einer Alarmliste werden die bevorstehenden Messungen aller aktiven Messserien verwaltet, und das Analysegerät zeigt dem Benutzer mittels stillen Alarms an, dass eine Messung durchzuführen ist.

Optional kann der Benutzer während der Messung ein Trend-Diagramm des aktuellen Patienten aufrufen. Das Trend-Diagramm zeigt den gemessenen Glukose Wert und die tatsächlich verabreichte Insulindosis für den gesamten Zeitraum der Messserie oder ein Teil dieses Zeitraums.

Weiters kann der Benutzer in der Datenbank ein Trend-Diagramm für einen beliebigen Patienten aufrufen.

Der im Analysegerät 1 implementierte Dosierungsvorschlagsalgorithmus ist beispielsweise eine erfindungsgemäße Fortbildung des "Glucommander"-Algorithmus, wie im eingangs erwähnten Dokument "Intravenous Insulin Infusion Therapy: Indications, Methods, and Transition to Subcutaneous Insulin Therapy", Bode et al, ENDOCRINE PRACTICE, Vol 10 (Suppl 2) March/April 2004 beschrieben.

Der "Glucommander"-Algorithmus beruht auf der Formel:

$$IR(k) = MM(k) \times (BG(k) - TH)$$

mit:

IR     Insulindosis [Einheiten pro Stunde]
k     Iterationsschritt [äquivalent zu Messzeitpunkten n, n+1, ..]
MM     Multiplikator ...
BG     Blutzuckerspiegel des Patienten
TH     Mindest-Blutzuckerschwelle, ab der Insulinverabreichung erfolgt, typisch auf 60 mg/dl festgelegt.

Der Multiplikator MM wird bei jedem Iterationsschritt neu festgelegt. Ein Anfangswert des Multiplikators MM für die erste Messung wird gewöhnlich auf 0,02. eingestellt. Für Folgemessungen kann der Arzt den Multiplikator mit einem "Agressivitätsfaktor," multiplizieren, der die Insulindosis mitdefiniert. Typische Werte dieses "Agressivitätsfaktors" sind mild 0.5, normal 1 oder variabel 0.5 bis 2. Die Iterationsschritte k, entsprechend dem Intervall TM zwischen zwei Messzeitpunkten n, n+1 (siehe Fig. 2) werden zunächst auf 30 Minuten festgelegt. Gemäß dem "Glucommander"-Algorithmus wird der Multiplikator MM jene Stunde am 0,01 nachgestellt um den Soll-Blutzuckerspiegel zu erreichen. Falls das Resultat eine Unterschreitung des Soll-Blutzuckerspiegels ist, so erfolgt eine Verringerung um 0,01; falls das Resultat ein Erreichen oder Beibehalten des Soll-Blutzuckerspiegels ist, so erfolgt keine Änderung; falls das Resultat über dem Soll-Blutzuckerspiegel ist und der Blutzuckerspiegel sich nicht um 25% reduziert hat, so erfolgt eine Erhöhung um 0,01. Details des "Glucommander"-Algorithmus können insbesondere Appendix 3 des zitierten Artikels von Bode et al entnommen werden.

Der "Glucommander"-Algorithmus setzt voraus, dass die Abstände zwischen den Iterationsschritten k exakt eingehalten werden. Solange dies möglich ist, arbeitet der erfindungsgemäße Dosierungsvorschlagsalgorithmus gemäß dem "Glucommander"-Algorithmus, wobei die Dosierungsempfehlung DS der Insulindosis IR in obiger Formel entspricht. Wie oben ausgeführt, ist es entweder gerätebedingt oder beriutzerbedingt nicht immer möglich, die Abstände zwischen den Iterationsschritten k exakt einzuhalten, d.h. es bestehen Messausschlusszeitfenster EX. Die vorliegende Erfindung bietet die besprochene Lösung für dieses Problem, indem die Messzeitpunkte außerhalb der zu Messausschlusszeitfenster EX verlegt werden (Fig. 2) oder die Messausschlusszeiten EX verlegt werden (Fig. 3). In der vorliegenden beispielhaften Implementierung des Dosierungsvorschlagsalgorithmus wird dies folgendermaßen gehandhabt:

Falls der vorläufige nächste Messzeitpunkt n+1 innerhalb des Messausschlusszeitfensters EX liegt, wird der nächste Messzeitpunkt nach vorne (n+1)' oder hinten (n+1)" verschoben, wie oben anhand der Fig. 2 erklärt worden ist, und zwar so, dass der Messzeitpunkt ein bestimmtes Zeitintervall, beispielsweise 5 Minuten, vom Beginn bzw. dem Ende des Zeitausschlussfensters EX entfernt ist.

Falls bei der Verlegung des Messzeitpunkts (n+1)' nach vorne ein minimales Zeitintervall TM , beispielsweise von weniger als 15 Minuten, unterschritten würde, so interpretiert die Risikoabschätzung des Dosierungsvorschlagsalgorithmus diese Zeitspanne als zu kurz, um bei der nächsten Messung eine verlässliche Aussage über die Veränderung des Blutzuckerspiegels des Patienten treffen zu können. In diesem Fall wird der nächste Messzeitpunkt (n+1)" auf funk Minuten hinter das Messausschlusszeitfenster EX verlegt und eine Waminformation (AL) an den Arzt abgegeben.

Für die Erläuterung eine weiteren implementierten Risikoabschätzung wird nun nochmals auf Fig. 4 Bezug genommen. Wie ersichtlich, würde der (lineare) Blützuckerverlauf BG(DS) bei einer Verlegung des nächsten Messzeitpunkts nach hinten (n+1)" zu einem Unterschreiten des Sollblutzuckerspiegels BGS um die Differenz, D" führen. Dies impliziert die Gefahr von Hypoglykämie des Patenten. Daher führt der Dosierungsvorschlagsalgorithmus, wenn er die drohende Unterschreitung des Sollblutzuckerspiegels BGS detektiert, eine nochmalige Berechnung einer adaptierten Empfehlung DS" unter Zugrundelegung des verlängerten Zeitintervalls zwischen dem Messzeitpunkt n und dem nächsten Messzeitpunkt (n+1)" durch. Die adaptierte Empfehlung DS" der Insulinverabreichung kann durch lineare Interpolation berechnet werden, die den linearen Blutzuckerspiegelverlauf BG(DS") zum Resultat hat.

## Patentansprüche

1. Verfahren zur Durchführung einer Serie von Messungen zumindest eines physiologischen Parameters, insbesondere Blutwertes, wie z.B. Blutzucker, an einem Patienten zu diskreten Messzeitpunkten (n) entnommenen Proben (S) und zur Errechnung einer Empfehlung (DS) für eine Dosierung zumindest eines dem Patienten bis zum nächsten Messzeitpunkt zu verabreichenden Wirkstoffs anhand eines den zumindest einen gemessenen physiologischen Parameter einbeziehenden Dosierungsvorschlagsalgorithmus, wodurch zumindest ein physiologischer Parameter des Patienten auf einen Zielbereich eingestellt wird, **dadurch gekennzeichnet, dass** der nächste Messzeitpunkt (n+1, (n+1)', (n+1)") unter Berücksichtigung von Messausschlusszeitfenstern (EX), in denen messgerätebedingt oder benutzerbedingt keine Messungen möglich sind, ermittelt wird, wobei der ermittelte nächste Messzeitpunkt (n+1, (n+1)', (n+1)") eine Variable des Dosierungsvorschlagsalgorithmus darstellt.

2. Verfahren nach Anspruch 1, **dadurch gekennzeichnet, dass** der Dosierungsvorschlagsalgorithmus Patientendaten, wie Gewicht, Ernährungsgewohnheiten, etc. bei der Errechnung der Empfehlung (DS) einbezieht.

3. Verfahren nach Anspruch 1 oder 2, **dadurch gekennzeichnet, dass** der nächste Messzeitpunkt ((n+1), (n+1)', (n+1)") ermittelt wird, indem zum letzten Messzeitpunkt (n) ein Zeitintervall (TM) addiert wird und falls der so errechnete Messzeitpunkt (n+1) in einem Messausschlusszeitfenster (EX) liegt, dieser Messzeitpunkt ( (n+1)', (n+1)") außerhalb des Messausschlusszeitfensters (EX) verlegt wird.

4. Verfahren nach Anspruch 3, **dadurch gekennzeichnet, dass** die Verlegung des nächsten Messzeitpunkts ((n+1)', (n+1)") außerhalb des Messausschlusszeitfensters (EX) unter Vorausberechnung eines zu erwartenden Messwerts, insbesondere durch Interpolation, und einer Risikoabschätzung, ob der zu erwartende Messwert für den Zustand des Patienten akzeptabel ist, erfolgt.

5. Verfahren nach Anspruch 3, **dadurch gekennzeichnet, dass** die Verlegung des nächsten Messzeitpunkts ((n+1)', (n+1)") außerhalb des Messausschlusszeitfensters (EX) unter Risikoabschätzung einer für den Zustand des Patienten maximal zulässigen Zeitspanne zwischen zwei Messzeitpunkten erfolgt.

**6.** Verfahren nach Anspruch 4 oder 5, **dadurch gekennzeichnet, dass** bei unvertretbar hohem Risiko eine Warninformation (AL) ausgegeben wird, wobei optional der Messzeitpunkt vor das Messausschlusszeitfenster verlegt wird.

**7.** Verfahren nach Anspruch 1 oder 2, wobei das Messausschlusszeitfenster durch Aktionen (CL1) des Analysegeräts bedingt ist, **dadurch gekennzeichnet, dass** der nächste Messzeitpunkt (n+1) ermittelt wird, indem zum letzten Messzeitpunkt (n) ein Zeitintervall (TM) addiert und geprüft wird, ob der sich daraus ergebende nächste Messzeitpunkt (n+1) in dem Messausschlusszeitfenster liegt und, falls zutreffend, das messgerätebedingte Messausschlusszeitfenster (EX) außerhalb des nächsten Messzeitpunkts (n+1) verlegt wird.

**8.** Verfahren nach Anspruch 1 oder 2, wobei das Messausschlusszeitfenster durch Aktionen (US1) des Benutzers bedingt ist, **dadurch gekennzeichnet, dass** der nächste Messzeitpunkt (n+1) ermittelt wird, indem zum letzten Messzeitpunkt (n) ein Zeitintervall (TM) addiert und geprüft wird, ob der sich daraus ergebende nächste Messzeitpunkt (n+1) in dem Messausschlusszeitfenster liegt und, falls zutreffend, dem Benutzer eine Meldung (INF) übermittelt wird, in der er aufgefordert wird, den Zeitpunkt für seine Aktionen (US1) und damit das benutzerbedingte Messausschlussfenster (EX) außerhalb des nächsten Messzeitpunkts (n+1) zu verlegen.

**9.** Verfahren nach Anspruch 8, **dadurch gekennzeichnet, dass** der Benutzer das benutzerbedingte Messausschlussfenster (EX) außerhalb des nächsten Messzeitpunkts (n+1) verlegt, und gegebenenfalls auch nachfolgende benutzerbedingte Messausschlussfenster verlegt.

**10.** Medizinisch-diagnostisches Analysegerät (1), wie beispielsweise ein Blutanalysegerät, zur Aufnahme von einem Patienten zu diskreten Messzeitpunkten entnommenen Proben (S), zumindest einem Messsensor (3) zur Messung zumindest eines physiologischen Parameter, insbesondere Blutwertes, wie z.B. Blutzucker, an den Proben, einer die Messsignale (MS) des zumindest einen Messsensors erhaltenden Recheneinheit (4) zur Aufbereitung von Messwerten des zumindest einen physiologischen Parameters aus den Messsignalen, zur Errechnung einer Empfehlung (DS) für eine Dosierung eines dem Patienten bis zum nächsten Messzeitpunkt zu verabreichenden Wirkstoffs anhand eines den zumindest einen gemessenen physiologischen Parameter einbeziehenden Dosierungsvorschlagsalgorithmus, wobei die Recheneinheit (4) dazu ausgebildet ist, das Verfahren nach einem der Ansprüche 1 bis 9 abzuarbeiten.

**11.** Analysegerät nach Anspruch 10, **gekennzeichnet durch** ein Ausgabeinterface (5), über das die Recheneinheit (4) die Dosierungsempfehlung (DS), Warnungen (AL) und andere Meldungen (INF) an einen Benutzer ausgibt.

**12.** Analysegerät nach Anspruch 10 oder 11, **dadurch gekennzeichnet, dass** die Recheneinheit dazu ausgebildet ist, das Verfahren parallel für eine Vielzahl von Patienten durchzuführen.

**Claims**

**1.** A method for performing a series of measurements of at least one physiological parameter, in particular a blood value, such as, e.g., blood sugar, in samples (S) taken from a patient at discrete measurement points (n) and for calculating a recommendation (DS) for a dose of a substance to be administered to the patient by the next measurement point by way of a dosing proposal algorithm taking into account the at least one measured physiological parameter, by means of which a physiological parameter of the patient is adjusted to a target area, **characterized in that** the next measurement point (n+1, (n+1)', (n+1)") is determined in consideration of measurement exclusion time windows (EX), in which measurements are not possible due to the measuring device or user, with the determined next measurement point (n+1, (n+1)', (n+1)") representing a variable of the dose proposing algorithm.

**2.** A method according to claim 1, **characterized in that** the dose proposal algorithm includes patient data such as weight, dietary habits, etc. in the calculation of the recommendation (DS).

**3.** A method according to claim 1 or 2, **characterized in that** the next measurement point (n+1, (n+1)', (n+1)") is determined, by adding a time interval (TM) to the last measurement point (n) and, if the measurement point (n+1) thus calculated is within a measurement exclusion time window (EX), by rescheduling this measurement point ((n+1)', (n+1)") out of the measurement exclusion time window (EX).

**4.** A method according to claim 3, **characterized in that** rescheduling of the next measurement point ((n+1)', (n+1)") out of the measurement exclusion time window (EX) is performed under pre-calculating a measurement value to be expected, in particular by interpolation, and a risk assessment, whether the measurement value to be expected is acceptable for the condition of the patient.

**5.** A method according to claim 3, **characterized in that** the re-scheduling of the next measurement point ((n+1)', (n+1)") out of the measurement exclusion time window (EX) is performed under risk assessment of a time interval between two measurement points that is maximal permissible for the condition of the patient.

6. A method according to claim 4 or 5, **characterized in that** in the case of an unjustifiable high risk there is given an alerting information (AL), wherein optionally the measurement point is re-scheduled before the measurement exclusion time window.

7. A method according to claim 1 or 2, wherein the measurement exclusion time window is determined by actions (CLS 1) of the analysis device, **characterized in that** the next measurement point (n+1) is determined, by adding a time interval (TM) to the last measurement point (n) and verifying whether the resulting next measurement point (n+1) is within the measurement exclusion time window and, if this is the case, re-scheduling the measuring device-specific measurement exclusion time window (EX) out of the next measurement point (n+1).

8. A method according to claim 1 or 2, wherein the measurement exclusion time window is determined by actions (US 1) by the user, **characterized in that** the next measurement point (n+1) is determined, by adding a time interval (TM) to the last measurement point (n) and verifying, whether the next measurement point (n+1) resulting thereof is within the measurement exclusion time window and, if this is the case, transmitting a message (INF) to the user asking him to re-schedule the point of time for his actions (US 1) and thus the user-specific measurement exclusion time window (EX) out of the next measurement point (n+1).

9. A method according to claim 8, **characterized in that** the user re-schedules the user-specific measurement exclusion time window (EX) out of the next measurement point (n+1) and optionally also re-schedules subsequent user-specific measurement exclusion time windows.

10. A medicinal-diagnostic analysis device (1), such as , for example, a blood analysis device, for recording samples (S) taken from a patient at discrete points of time, having at least one measuring sensor (3) for measuring at least one physiological parameter, in particular a blood value, such as, e.g., blood sugar, in the samples, a computing unit (4) receiving the measurement signals (MS) from the at least one measuring sensor for processing measurement values of the at least one physiological parameter from the measurement signals, for calculating a recommendation (DS) for a dose of a substance to be administered to a patient by the next measurement point by way of a dose proposing algorithm taking into account the at least one measured physiological parameter, wherein the computing unit (4) is adapted to perform the method according to any of the claims 1 to 9.

11. An analysis device according to claim 10, **characterized by** an output interface (5), via which the computing unit (4) outputs the dose recommendation (DS), alerting information (AL) and other information (INF) to a user.

12. An analysis device according to claim 10 or 11, **characterized in that** the computing unit is adapted to perform the method in parallel for a plurality of patients.

## Revendications

1. Procédé destiné à effectuer une série de mesures d'au moins un paramètre physiologique, en particulier d'une valeur sanguine, comme p. ex. la glycémie, sur des échantillons (S) prélevés à des moments de mesure discrets (n) sur un patient, et à calculer une recommandation (DS) pour un dosage d'au moins un principe actif à administrer au patient jusqu'au moment de mesure suivant à l'aide d'un algorithme de proposition de dosage incluant ledit au moins un paramètre physiologique mesuré, permettant ainsi d'ajuster au moins un paramètre physiologique du patient à une plage ciblée, **caractérisé en ce que** le moment de mesure suivant (n+1, (n+1)', (n+1) ") est déterminé en tenant compte de fenêtres temporelles d'exclusion de mesure (EX) dans lesquelles, pour des raisons liées à l'appareil de mesure ou à l'utilisateur, aucune mesure n'est possible, le moment de mesure suivant déterminé (n+1, (n+1)', (n+1) ") représentant une variable de l'algorithme de proposition de dosage.

2. Procédé selon la revendication 1, **caractérisé en ce que** l'algorithme de proposition de dosage inclut des données du patient, telles que le poids, les habitudes alimentaires, etc. lors du calcul de la recommandation (DS).

3. Procédé selon la revendication 1 ou 2, **caractérisé en ce que** le moment de mesure suivant ((n+1, (n+1)', (n+1) ") est déterminé en additionnant au dernier moment de mesure (n) un intervalle de temps (TM) et si le moment de mesure ainsi calculé (n+1) se situe dans une fenêtre temporelle d'exclusion de mesure (EX), ce moment de mesure ((n+1)', (n+1) ") est déplacé à l'extérieur de la fenêtre temporelle d'exclusion de mesure (EX).

4. Procédé selon la revendication 3, **caractérisé en**

**ce que** le déplacement du moment de mesure suivant ((n+1)', (n+1) ") à l'extérieur de la fenêtre temporelle d'exclusion de mesure (EX) est effectué en calculant d'avance une valeur de mesure escomptée, en particulier par interpolation, et en évaluant le risque que la valeur de mesure escomptée soit acceptable pour l'état du patient.

**5.** Procédé selon la revendication 3, **caractérisé en ce que** le déplacement du moment de mesure suivant ((n+1)', (n+1) ") à l'extérieur de la fenêtre temporelle d'exclusion de mesure (EX) est effectué en évaluant le risque d'un intervalle de temps maximum admissible pour l'état du patient entre deux moments de mesure.

**6.** Procédé selon la revendication 4 ou 5, **caractérisé en ce qu'**en cas de risque inacceptablement élevé, une information d'avertissement (AL) est émise, et optionnellement le moment de mesure est déplacé en amont de la fenêtre temporelle d'exclusion de mesure.

**7.** Procédé selon la revendication 1 ou 2, dans lequel la fenêtre temporelle d'exclusion de mesure est conditionnée par des actions (CL1) de l'appareil d'analyse, **caractérisé en ce que** le moment de mesure suivant (n+1) est déterminé en additionnant au dernier moment de mesure (n) un intervalle de temps (TM) et en vérifiant si le moment de mesure suivant (n+1) ainsi obtenu se situe dans la fenêtre temporelle d'exclusion de mesure et, si tel est le cas, la fenêtre temporelle d'exclusion de mesure (EX) conditionnée par l'appareil de mesure est déplacée à l'extérieur du moment de mesure suivant (n+1).

**8.** Procédé selon la revendication 1 ou 2, dans lequel la fenêtre temporelle d'exclusion de mesure est conditionnée par des actions (US1) de l'utilisateur, **caractérisé en ce que** le moment de mesure suivant (n+1) est déterminé en additionnant au dernier moment de mesure (n) un intervalle de temps (TM) et en vérifiant si le moment de mesure suivant (n+1) ainsi obtenu se situe dans la fenêtre temporelle d'exclusion de mesure et, si tel est le cas, une information (INF) est transmise à l'utilisateur, dans laquelle celui-ci est invité à déplacer le moment de ses actions (US1), et donc la fenêtre d'exclusion de mesure (EX) conditionnée par l'utilisateur, à l'extérieur du moment de mesure suivant (n+1 ).

**9.** Procédé selon la revendication 8, **caractérisé en ce que** l'utilisateur déplace la fenêtre d'exclusion de mesure (EX) conditionnée par l'utilisateur à l'extérieur du moment de mesure suivant (n+1), et le cas échéant il déplace aussi les fenêtres d'exclusion de mesure conditionnée par l'utilisateur suivantes.

**10.** Appareil d'analyse médico-diagnostique (1), comme par exemple un appareil d'analyse sanguine, destiné à recevoir des échantillons (S) prélevés à des moments de mesure discrets sur un patient, comprenant au moins un capteur de mesure (3) destiné à mesurer au moins un paramètre physiologique, en particulier une valeur sanguine, comme p. ex. la glycémie, sur lesdits échantillons, une unité de calcul (4) recevant les signaux de mesure (MS) dudit au moins un capteur de mesure, destinée à traiter des valeurs mesurées dudit au moins un paramètre physiologique à partir des signaux de mesure, afin de calculer une recommandation (DS) pour un dosage d'un principe actif à administrer au patient jusqu'au moment de mesure suivant à l'aide d'un algorithme de proposition de dosage incluant ledit au moins un paramètre physiologique mesuré, l'unité de calcul (4) étant conçue pour exécuter le procédé selon l'une des revendications 1 à 9.

**11.** Appareil d'analyse selon la revendication 10, **caractérisé par** une interface de sortie (5) via laquelle l'unité de calcul (4) émet la recommandation de dosage (DS), des avertissements (AL) et d'autres informations (INF) à un utilisateur.

**12.** Appareil d'analyse selon la revendication 10 ou 11, **caractérisé en ce que** ladite unité de calcul est conçue pour mettre en oeuvre le procédé d'une manière parallèle pour une pluralité de patients.

Fig. 1

Fig. 2

Fig. 3

Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2009049245 A
- US 20070168136 A
- WO 2008057213 A

**In der Beschreibung aufgeführte Nicht-Patentliteratur**

- **Bode et al.** Intravenous Insulin Infusion Therapy: Indications, Methods, and Transition to Subcutaneous Insulin Therapy. *ENDOCRINE PRACTICE,* Marz 2004, vol. 10 (2
- **Davidson et al.** *Diabetes Care,* 2005, vol. 20 (10), 2418-2423